# EUROPEAN PATENT APPLICATION

(11) **EP 2 749 349 A1**
(43) Date of publication of application: **02.07.2014**
(21) Application number: 12825200.4
(22) Date of filing: 21.08.2012
(51) Int. Cl.: B01J 19/00, C12M 1/00, C12M 1/38, G01N 35/00, G01N 35/08, G01N 37/00, B01L 3/00, B01L 7/00

(54) **MICRO FLUID DEVICE**

(30) Priority: 22.08.2011 JP 2011180318
(71) Applicant: Panasonic Corporation, Osaka 571-8501 (JP)
(72) Inventor: TACHIBANA, Hiroaki, Chuo-ku Osaka 540-6207 (JP); TSUJI, Koji, Chuo-ku Osaka 540-6207 (JP); SAIJO, Takashi, Chuo-ku Osaka 540-6207 (JP); TAMIYA, Eiichi, Osaka 565-0871 (JP); SAITO, Masato, Osaka 565-0871 (JP)
(74) Representative: Müller-Boré & Partner Patentanwälte PartG mbB
(86) International application number: PCT/JP2012/005236
(87) International publication number: WO 2013/027393

(57) **Abstract**

To provide a microfluidic device which does not apply a meandering reaction channel and thus can be reduced in size, the microfluidic device comprising a reaction channel is characterized in that a plurality of thermal cycle regions which respectively comprise at least two thermal regions with different temperatures are repeatedly provided and the reaction channel passes through the plurality of thermal cycle regions.

## Description

### Technical Field

The present invention relates to a microfluidic (or micro-fluid) device. More specifically, the present invention relates to the microfluidic device such as a microreactor, an integrated DNA device, and a micro-electrophoresis device.

### Background Art

In recent years, micromachining techniques have been used in not only electrical and electronic fields but also any other field such as fluidics, mechanics and optics, and are the object of active researches and developments. Furthermore, it would not be an exaggeration to say that the micromachining techniques will draw greater attention in the future. Development of micromachining techniques has enabled downsizing of devices, which surely meets the social demands for resource saving and energy saving.

In particular, the microfluidic device, which should be typical products of micromachining techniques, is considered to be of great use in the fields of the microreactor, integrated DNA devices and micro-electrophoresis devices, since the microfluidic device allow reaction solution to cause reaction using extremely small amount of sample or regent.

Furthermore, increased surface area associated with the miniaturization allows for rapid heat transfer, which is expected to be advantageous for microfluidic device which requires desired temperature control of reaction fluid. Such microfluidic device is disclosed in Patent Document 1, which has a meandering reaction channel.

Patent Document 1 discloses a microchemical device which can selectively amplify a certain part of a DNA (deoxyribonucleic acid) by allowing reaction solution to flow through three different thermal regions repeatedly at least for 5 to 40 cycles as represented in PCR (polymerase chain reaction) method. To allow the reaction solution to flow through three different thermal regions repeatedly at least for 5 to 40 cycles, its reaction channel has a meandering pattern.

### Prior Art Documents

### Patent Document

Patent Document 1: JP 2002-18271 A

### Summary of Invention

### Problems to be solved by the Invention

However, since reaction channel of conventional microfluidic device has a meandering pattern, the conventional microfluidic device has a problem with difficulty in reducing the device size.

Therefore, it is an object of the present invention to provide a microfluidic device which does not apply a meandering reaction channel and thus can be reduced in size.

### Means for Solving the Problems

To solve the above problem,
a microfluidic device comprising a reaction channel is characterized in that a plurality of thermal cycle regions which respectively comprise at least two thermal regions with different temperatures are repeatedly provided, and the reaction channel passes through the plurality of thermal cycle regions.

According to a preferred aspect to solve the above problem,
the microfluidic device further comprises a heater unit, wherein the heater unit comprises a plurality of heaters which are set at different temperatures, and
the thermal regions are formed around the heaters.

According to another preferred embodiment,
the microfluidic device further comprises a substrate and a cover plate which are bonded to each other,
wherein the heater unit is placed on at least either one of the substrate and the cover plate, and
the reaction channel is formed between the substrate and the cover plate.

According to another preferred embodiment,
at least two of the reaction channels are arranged in parallel.

According to another preferred embodiment,
the at least two of the reaction channels arranged in parallel share the same heater unit.

According to another preferred embodiment,
the microfluidic device further comprises a sample injection port and a sample discharge port,
wherein the at least two of the reaction channels arranged in parallel are connected to the same sample injection port.

According to another preferred embodiment,
the heaters are metal thin film heaters.

According to another preferred embodiment,

The microfluidic device further comprises a detection electrode to measure a concentration of a reaction fluid flowing in the reaction channel.

According to another preferred embodiment,
the microfluidic device further comprises a reaction reagent which is held at a position between the sample injection port and the reaction channel which passes through the plurality of thermal cycle regions.

According to another preferred embodiment,
the microfluidic devices are arranged in series or in parallel or in a combination thereof.

### Advantageous Effect of Invention

The microfluidic device of the present invention comprises repetitive thermal cycle regions and a reaction channel is formed to pass through the plurality of thermal cycle regions. Thus, it is not required to form the reaction channel in a meandering pattern, which allows for reducing the size of the microfluidic device.

Furthermore, since it is not required to form the reaction channel in a meandering pattern, a plurality of reaction channels can be disposed in parallel in a single microfluidic device.

### Brief Description of the Drawings

Fig. 1 is an overall perspective view of a microfluidic device of the present invention.
Fig. 2 is an exploded perspective view of a microfluidic device of the present invention.
Fig. 3 is a cross sectional view illustrating a fabricating process of a substrate of a microfluidic device of the present invention.
Fig. 4 is a cross sectional view illustrating a fabricating process of a cover plate of a microfluidic device of the present invention.
Fig. 5 is a cross sectional view illustrating a bonding process of the cover plate to the substrate of the microfluidic device of the present invention.

### Best Mode for Carrying Out the Invention

The microfluidic device of the present invention will be described below.

In the following description, a microfluidic device is referred to as a "device". Further, the term "reaction fluid" as used herein refers to, but is not particularly limited to, liquid, gas or the mixture thereof which can flow through a reaction channel. For example, the term "fluid" as used herein also includes slurry, in which solid particles are dispersed in liquid.

In comparison to the conventional techniques in which a reaction channel is meandering so that reaction fluid can repeatedly pass though different thermal regions for multiple times, the device of the present invention is characterized in that it comprises repeated thermal cycle regions which comprise at least two thermal regions with different temperatures from each other.

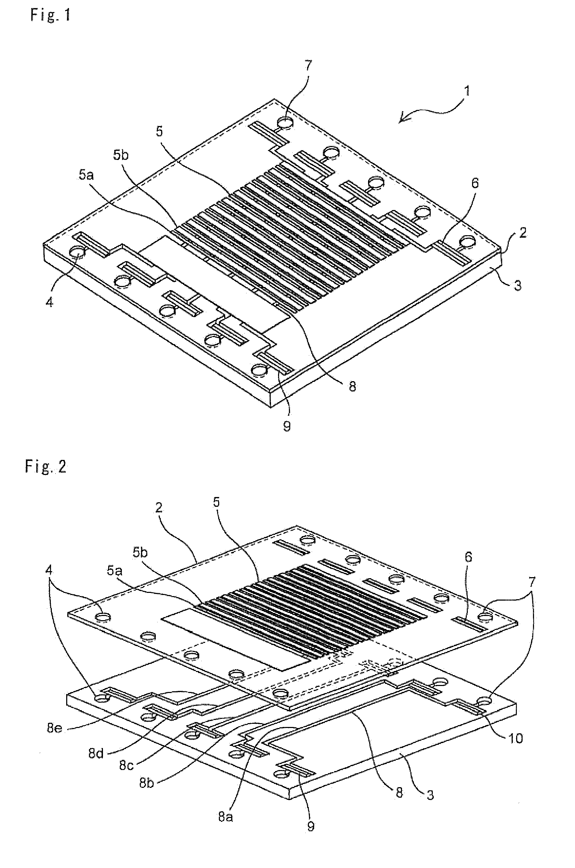

A device according to an embodiment of the present invention will be described in detail below. Fig. 1 is an overall perspective view of the device 1 of the present invention. Fig. 2 is an exploded perspective view of the device 1 of the present invention. The device 1 of the present invention is composed of a cover plate 2 and a substrate 3. The cover plate 2 comprises through holes which define a part of sample injection ports 4 and sample discharge ports 7, a heater unit 5 and detection electrodes 6. The heater unit 5 and the detection electrodes 6 are formed on the lower face of the cover plate 2. The heater unit 5 comprises heaters 5a and heaters 5b in an alternate arrangement, in which the heaters 5a and heaters 5b are set at different temperatures from each other. The through holes which define a part of the sample injection ports 4 and sample discharge ports 7 are formed to penetrate the plate from the upper face to lower face. The substrate 3 comprises recesses which define a part of the sample injection ports 4, recesses which define a part of the sample discharge ports 7, reaction channels 8, reaction reagent reservoirs 9 and detecting sections 10. The reaction channels 8 are located between the reaction reagent reservoirs 9 and the detecting sections 10, and are defined between channels 14 formed on the substrate 3 and the cover plate 2. The reaction channels 8 comprise reaction channels 8a, 8b, 8c, 8d and 8e in the device 1. The reaction channel 8c passes straight from the reaction regent reservoirs 9 to the detecting sections 10. The reaction channel 8d has a symmetrical pattern to the reaction channel 8b. The reaction channel 8e has a symmetrical pattern to the reaction channel 8a. The reaction channels 8a, 8b, 8c, 8d and 8e are located to pass below the heater unit 5. The lower face of the cover plate 2 and the upper face of the substrate 3 with the above-mentioned structure are bonded to each other such that: the through holes of the sample injection ports 4 on the cover plate 2 meet the recesses on the substrate 3 which define a part of the sample injection ports 4; the through holes of the sample discharge ports 7 on the cover plate 2 meet the recesses of the sample discharge ports 7 on the substrate 3; and the detection electrodes 6 are located over the discharge sections 10.

In the heater unit 5, the heaters 5a and heaters 5b set at different temperature from each other form thermal regions around each heater. A temperature region refers to a certain temperature zone formed around a heater. The thermal regions formed around each heater are thermal regions with different temperatures from each other. In the embodiment, two thermal regions with different temperatures are alternately formed in the direction from the sample injection ports 4 to the sample discharge ports 7. When the two thermal regions with different temperatures from each other are alternately formed in the direction from the sample injection ports 4 to the sample discharge ports 7, it can be considered that a plurality of thermal cycle regions which are composed of two thermal regions with different temperatures from each other are repeatedly formed in the direction from the sample injection ports 4 to the sample discharge ports 7.

The substrate 3 is made of, for example, silicon. The cover plate 2 is made of glass. The sample injection ports 4 and the sample discharge ports 7 have, for example, a circular cross-sectional shape. However, their cross-sectional shape is not limited to a circular shape. For example, it may be oval, rectangular, square or polygonal shape. The reaction channels 8 serve as channels through which reaction fluid flows from the reaction reagent reservoirs 9 to the detecting sections 10, and pass (or extend) through the plurality of repeated thermal cycle regions. The reaction channels 8 passes straight at the part within the plurality of repeated thermal cycle regions. The sample injection ports 4 are inlets for injecting the reaction fluid from the outside, and are preferably placed at a side end of the device 1. The sample discharge ports 7 are located opposite to the sample injection ports 4. The reaction reagent reservoirs 9 are located between the sample injection ports 4 and the reaction channels 8, and are loaded with a reaction reagent which is held in a dry condition. The detecting sections 10 are provided so that the detection electrodes 6 can measure the concentration of the reaction fluid. The detection electrodes 6 are located between the heater unit 5 and the sample discharge ports 7. The detection electrodes 6 are thin film electrodes for measuring the concentration of the reaction fluid by applying a voltage and detecting a current value. The sample discharge ports 7 are outlets from which the reaction fluid in the device 1 is discharged to the outside.

Next, the reaction process of the reaction fluid in the device 1 of the present invention will be described. First, desired reaction solution is injected into the sample injection ports 4, and then the reaction solution mixes with the reaction reagent which is previously loaded in the reaction reagent reservoirs 9. By virtue of the reaction reagent reservoirs 9, the device 1 of the present invention allows for rapid reaction with the reaction reagent without using an external reactor. Then, the reaction fluid which is the mixture of the reaction reagent and the reaction solution advances to the reaction channels 8. Then, the reaction fluid in the reaction channels 8 advances through the plurality of repeated thermal cycle regions toward the detecting sections 10. Then, after the reaction fluid has undergone reaction by passing through the plurality of thermal cycle regions, it advances to the detecting sections 10. In the detecting sections 10, the concentration of the reaction fluid is measured by applying a voltage on the detection electrodes 6 and detecting a current value. Lastly, the measured reaction fluid is discharged from the sample discharge ports 7.

A method for manufacturing the device 1 according to the embodiment of the present invention will be described below.

### (Fabrication of Substrate 3)

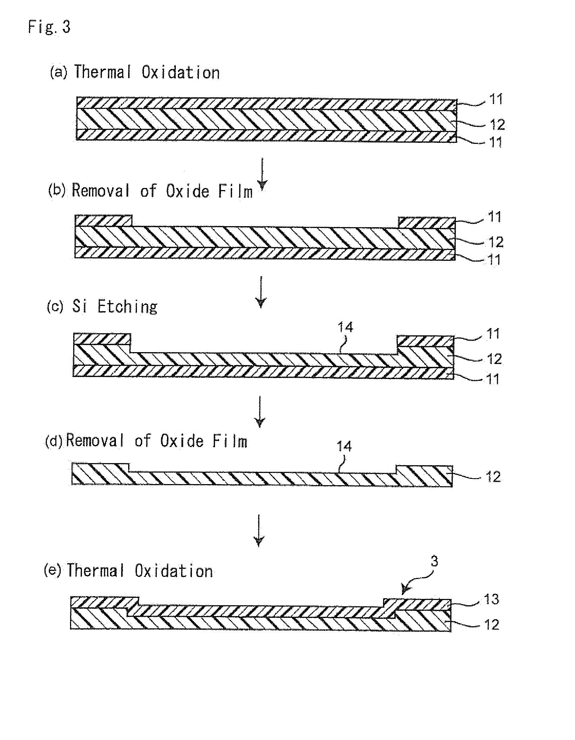

Fig. 3 is a cross sectional view illustrating a fabricating process of the substrate 3 of the device 1 of the present invention. First, in a diffusion furnace with an oxygen and hydrogen atmosphere, an oxide film 11 is formed on the both faces of a silicon substrate 12 (Fig. 3(a)). Then, a resist is formed on the oxide film 11 by photolithography, and the oxide film 11 is etched by reactive ion etching (RIE) (Fig. 3(b)). Then, the resist is removed, and the part where silicon is exposed is etched to form the channels 14 which include the recesses defining a part of the sample injection ports 4, the reaction reagent reservoirs 9, the reaction channels 8, the detecting sections 10 and the recesses defining a part of the sample discharge ports 7 (Fig. 3(c)). Then, the oxide film 11 on the both faces is removed by etching with hydrofluoric acid or the like (Fig. 3(d)). Lastly, an oxide film 13 is formed by thermal oxidation to enhance the hydrophilicity of the reaction channels 8 (Fig. 3(e)). The substrate 3 is thus obtained. Besides the above-mentioned method, if the substrate 3 is made of material other than silicon, such as resin for example, the channels 14 may be formed by injection molding or the like.

### (Fabrication of Cover Plate 2)

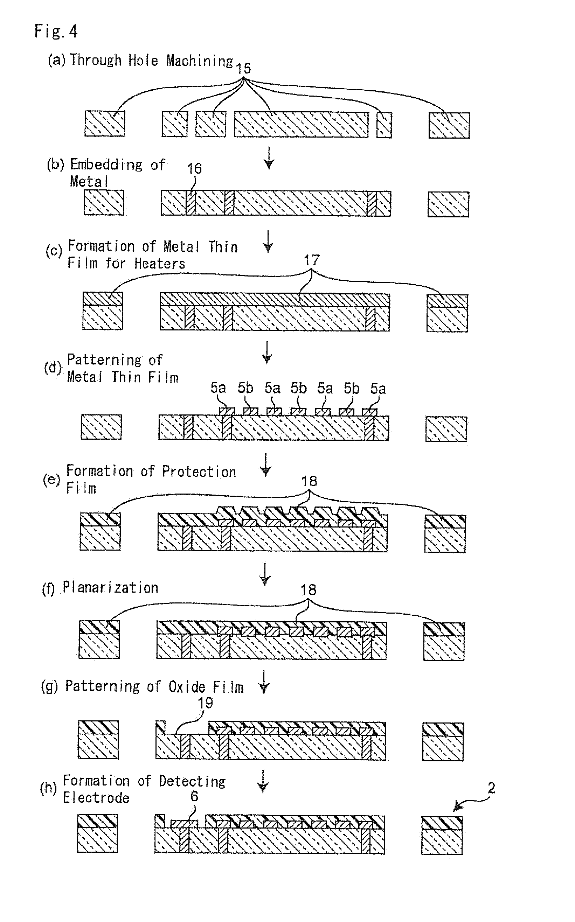

Fig. 4 is a cross sectional view illustrating a fabrication process of the cover plate 2 of the device 1 of the present invention. First, the through holes are formed on the glass plate 15 by sandblasting or drilling (Fig. 4(a)). Then, the through holes are filled with metals 16 such as copper by plating or the like (Fig. 4(b)). Then, a metal thin film 17 such as aluminum thin film for the heaters is formed by sputtering. In the present invention, the metal film may be of any kind. For example, aluminum, gold, platinum and the like are preferred (Fig. 4(c)). Then, the aluminum film formed by sputtering is patterned by photolithography and etching (Fig. 4(d)). The resulting metal film corresponds to the heaters 5a and 5b of the device 1 of the present invention. Then, in order to prevent the aluminum film to serve as the heaters 5a and 5b from being directly exposed to the reaction fluid, a silicon oxide film 18 is formed by plasma CVD (Fig. 4(e)). Then, the formed oxide film 18 is planarized by chemical mechanical polishing(CMP) (Fig. 4(f)). Then, to dispose the detection electrodes 6, the oxide film 18 is removed by photolithography and etching with hydrofluoric acid to form the bases 19 for the detection electrodes 6 (Fig. 4(g)). Lastly, gold or platinum thin film is formed by sputtering to form the detection electrodes 6 (Fig. 4(h)). The cover plate 2 is thus obtained.

### (Bonding of Substrate 3 and Cover Plate 2)

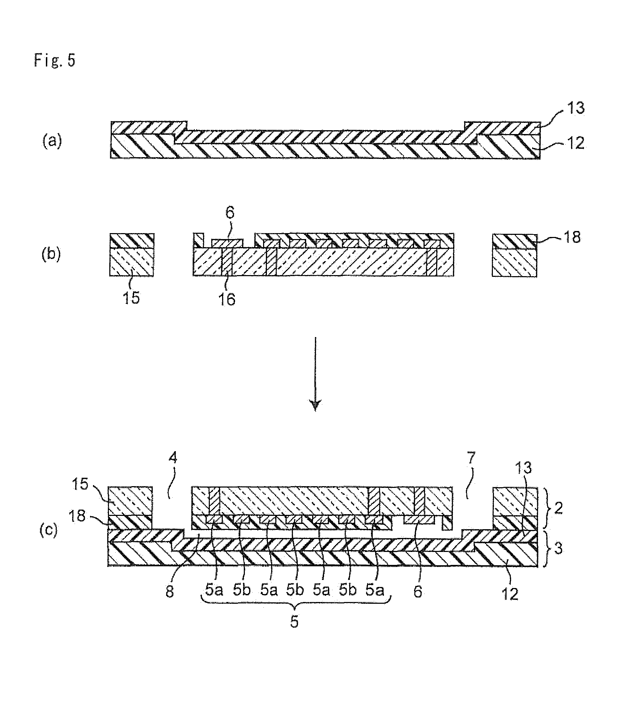

Fig. 5 is a cross sectional view illustrating a bonding process of the substrate 3 and the cover plate 2 of the device 1 of the present invention. First, the cover plate 2 (Fig. 5(b)) as illustrated in Fig. 4(h) is flipped over to prepare it as an upper plate, while the substrate 3 (Fig. 5(a)) as illustrated in Fig. 3(e) is prepared as a lower plate. Then, both plates are mated with each other, and then are heated up to approximately from 300°C to 400°C and maintained at the temperature. Then, when the temperature of the both plates reaches a desired temperature, a voltage of from 400 V to 800 V is applied to the cover plate 2. The applied voltage is maintained for from 20 to 60 minutes to bond the cover plate 2 to the substrate 3 (Fig. 5(c)). The device 1 of the present invention is thus obtained. The bonding method is not limited to anodic bonding. For example, the bonding process can be performed at ambient temperatures by employing surface activated bonding or the like.

The structure and manufacturing method of the device 1 of the present invention as well as the reaction process of the reaction fluid in the device 1 of the present invention have been described. The structure according to the above embodiments of the device 1 of the present invention has expanded the availability of the reaction channels 8 of the device 1 in various ways.

In the device 1 of the present invention, since the thermal cycle regions are repeated as many as the required number of thermal cycles, it is not required to form the reaction channels 8 in a meandering pattern. Furthermore, since it is not required to form the reaction channels 8 in a meandering pattern, at least two reaction channels 8 can be disposed in parallel in the single device 1, and the overall size of the device 1 can also be reduced. The shape of the reaction channels 8 in the device 1 may be the same or different from each other.

Further, since the reaction channels 8 in the device 1 of the present invention pass through the plurality of repeated thermal cycle regions, it is possible to design the reaction channels 8 not to go back toward the sample injection ports 4 but to extend in the direction from the sample injection ports 4 to the sample discharge ports 7. Thus, this can reduce the flow resistance of the reaction fluid.

Further, since the reaction channels 8 in the device 1 of the present invention pass through the plurality of repeated thermal cycle regions, the reaction channels 8 can pass straight from the reaction reagent reservoirs 9 to the detecting sections 10. This can minimize the flow resistance, as well as optimally stabilize uniform flow of the reaction fluid in the reaction channels 8.

Further, the single heater unit 5 can be shared by at least two reaction channels 8 which are arranged in parallel. This allows a plurality of reaction fluids to undergo reaction in the same temperature change condition, and thus it is possible to objectively observe differences in reaction behavior depending on the difference of the reaction fluids and to uniform heat transfer to the reaction fluids.

The device 1 of the present invention is not limited to the above-mentioned embodiment, and for example, may be the following embodiments.

In the device 1 of the present invention, the heaters in the heater unit 5 are set at two different temperatures from each other. However, the setting of the heaters is not limited to two different temperatures, and they may be set at three different temperatures from each other. For example, if the heaters in the heater unit 5 are set at three different temperatures from each other, the heaters of the heater unit 5 set at three different temperatures form three thermal regions with different temperatures around each heater, as with the case where they are set at two different temperatures from each other. These thermal regions with three different temperatures from each other are alternately arranged in the direction from the sample injection ports 4 to the sample discharge ports 7. When these three thermal regions with different temperatures from each other are repeatedly formed in the direction from the sample injection ports 4 to the sample discharge ports 7, it can be considered that a plurality of thermal cycle regions which are composed of three thermal regions with different temperatures from each other are repeatedly formed in the direction from the sample injection ports 4 to the sample discharge ports 7.

Further, the thermal cycle regions in the device 1 of the present invention are arranged repeatedly in succession. However, such repeated and successive arrangement is not essential, and another temperature region may be formed between two thermal cycle regions.

The position or size of the heaters may be changed so as to change the reaction behavior of the reaction fluid in the reaction channels 8 which flow through each of thermal regions. The temperature of the heaters does not have to sequentially ascend or descend in the order toward the sample discharge ports 7, and may be arranged in any combination. The heaters do not have to be placed apart from each other. For example, a single heater may be placed in a U-shape.

While the heater unit 5 is placed on the cover plate 2, it does not have to be placed on the cover plate 2 and may be placed on the substrate 3. Further, the sample injection ports 4 and the sample discharge ports 7 may be formed only on the substrate 3. Further, the number of the heater unit 5 does not have to be one, and may be two or more. For example, two different heater units 5 may be shared by at least two reaction channels 8 which are arranged in parallel.

The part of the reaction channels 8 which pass through the plurality of thermal cycle regions does not have to be straight, and may be curved such as in a semicircular or semioval shape. This can reduce the flow resistance of the reaction fluid, and thus it is possible to uniform a stream of the reaction fluid in the reaction channels 8. Further, it is preferred that the reaction channels 8 intersect the thermal cycle regions at a right angle.

At least two reaction channels 8 which are arranged in parallel may be connected to the same sample injection port 4. With this structure, the same reaction fluid flows in at least two reaction channels 8 and thus measurements obtained is more reliable.

The number of the sample injection ports 4 and the number of the sample discharge ports 7 may be changed according to the number of the reaction channels 8 which are arranged in parallel. Further, the number of the sample injection ports 4 and the number of the sample discharge ports 7 may be each the same or different from the number of the reaction channels 8 which are arranged in parallel.

In view of the foregoing, since the reaction channels 8 are formed in the device 1 such that they pass through the plurality of repeated thermal cycle regions, the device 1 is advantageous in that:
(1) the reaction channels 8 do not have to be formed in a meandering pattern;
(2) since the reaction channels 8 do not have to be formed in a meandering pattern, a plurality of reaction channels 8 can be effectively disposed in parallel in a single device 1;
(3) the device allows for stable flow of the reaction fluid and thus a flow control and a flow operation of the reaction fluid can be facilitated; and
(4) the device 1 can be reduced in size.

At least two pieces of the devices 1 may be arranged together. In this case, the arrangement of the devices 1 is not limited to parallel arrangement, and they may be arranged in series and/or in parallel, and/or any combination thereof. Such arrangements are advantageous when multiple different reaction processes are required while keeping the reduced size of each device 1.

Further, it would be easily understood by a skilled person in the art that the device of the present invention may be combined with other devices or the like with different functions and configurations. An example of such devices may be a syringe pump for injecting a sample to the device 1 of the present invention and/or for discharging the sample from the device 1 of the present invention. The discharge pressure of such syringe pumps is preferably from 0.1 MPa to 10 MPa.

### Examples

In this example, PCR is performed in the device. PCR is a reaction which amplifies the number of a DNA (deoxyribonucleic acid). Specifically, reaction solution includes a DNA fragment to be amplified, a polymerase and primers which serve as seeds of the DNA amplification, and this reaction solution undergoes the denaturation temperature (approx. 95°C), annealing temperature (approx. 60°C) and elongation temperature (approx. from 60°C to 75°C) repeatedly for approximately from 30 to 50 cycles so as to allow the DNA to increase in an exponential manner.

In the device 1, the heaters form high thermal regions for denaturation (e.g. from 95°C to 100°C) and low thermal regions for annealing and elongation (e.g. from 50°C to 75°C). Annealing and elongation are performed at the same temperature in this case, but they may be performed at different temperatures from each other. Thus, thermal regions for annealing and elongation may be separately formed. The retention time in each temperature region is adjusted by controlling the flow rate of the reaction fluid. The flow rate of the reaction fluid is controlled by changing the width and depth of the reaction channels 8. To flow the reaction fluid at a high flow rate, it is preferred that the retention time of the reaction fluid in each temperature region is, for example, approximately from 1 to 5 seconds at the high thermal regions and approximately from 4 to 20 seconds at the low thermal regions. To reduce the size, it is preferred that the fluid is flown by capillary action. For example, a desired flow rate is obtained when the reaction channels 8 are designed to be in the range from 20 µm to 100 µm in width and depth.

The reaction solution injected from the sample injection ports 4 reaches the reaction reagent holding sections 9 to dissolve the reaction reagent. The reaction reagent comprises the polymerase and primers, and is held in a dry condition such as by lyophilization. The reaction fluid in which the reaction reagent is dissolved is transferred in the reaction channels 8 by capillary action and then it passes through the high thermal regions and low thermal regions repeatedly so that PCR is performed. The resulting reaction fluid reaches the detecting sections 10 and thus the concentration of the amplified DNA can be measured from the interaction with the electrodes.

### (Fabricating Process of Substrate 3)

A fabricating process of the substrate 3 of the device 1 will be described. In this example, silicon is used for the substrate 3.

### (1) Thermal Oxidation

In a diffusion furnace with an oxygen and hydrogen atmosphere, the silicon oxide film 11 of approximately from 500 nm to 1 µm thick is formed on both faces of the silicon substrate 12.

### (2) Removal of Silicon Oxide Film 11

On the silicon oxide film 11, a resist is patterned by photolithography, and the silicon oxide film 11 is etched by reactive ion etching (RIE).

### (3) Si Etching

The resist is removed and the part of the exposed silicon is etched by deep reactive ion etching (DRIE), for example to a depth of 20 µm to 100 µm, so as to form the channels 14 which include the recesses defining a part of the sample injection ports 4, the reaction reagent reservoirs 9, the reaction channels 8, the detecting sections 10, and the recesses defining a part of the sample discharge ports 7 of the device. This silicon etching is not limited to DRIE, and may also be wet etching using TMAH or the like.

### (4) Removal of Silicon Oxide Film 11

The silicon oxide film 11 on both faces is removed by etching with hydrofluoric acid or the like.

### (5) Thermal Oxidation

To enhance the hydrophilicity of the reaction channels 8, an oxide film of approximately from 50 nm to 100 nm thick is formed.

### (Fabrication Process of Cover Plate 2)

A fabrication process of the cover plate 2 of the device 1 of the present invention will be described. In this example, glass is used for the cover plate 2.

### (1) Through Hole Machining

Through holes are formed on a glass plate 15 by sandblasting, drilling or the like.

### (2) Embedding of Metal 16

The through holes are filled with metal 16 such as copper by plating or the like.

### (3) Formation of Metal Thin Film of Heaters

A metal thin film 17 such as aluminum thin film of approximately from 0.5 µm to 1.5 µm for the heaters is formed by sputtering. The metal thin film 17 may be of any kind in the present invention. For example, aluminum, gold, platinum and the like are preferred.

### (4) Patterning of Metal Thin Film 17

The aluminum film formed by sputtering is patterned by photolithography and etching.

### (5) Formation of Protection Film

The silicon oxide film 18 of approximately from 1 µm to 2 µm thick is formed by plasma CVD. This prevents the aluminum thin film to serve as the heaters from being directly exposed to the fluid.

### (6) Planarization

The formed silicon oxide film 18 is planarized by chemical mechanical polishing (CMP).

### (7) Patterning of Oxide Film

At the part of the oxide film where the detection electrodes 6 are to be formed, the oxide film is removed by photolithography and etching with hydrofluoric acid.

### (8) Formation of Detecting Electrode 6

As the detection electrodes 6, a gold or platinum thin film is formed by sputtering.

### (Bonding of Substrate 3 and Cover Plate 2)

A bonding process of the cover plate 2 to the substrate 3 of the device 1 of the present invention will be described.

The bonding may be performed, for example, by anode bonding. First, the substrate 3 is mated with the cover plate 2, and heated up to approximately from 300°C to 400°C and maintained at the temperature. Then, when the temperature of the substrate 3 and cover plate 2 reaches a desired temperature, a voltage of from 400 V to 800 V with respect to the substrate 3 is applied to the cover plate 2. The applied voltage is maintained for 20 to 60 minutes, and thus a good bonding is obtained. The bonding method is not limited to anode bonding. For example, the bonding process can be performed at ambient temperatures by surface activated bonding or the like.

While the description has been made of the case where silicon and glass is used respectively for the substrate 3 and the cover plate 2 in this embodiment, the device of the present invention is not limited thereto and they may be made of, for example, an imprinted resin.

### Description of Reference Numerals

1 Microfluidic device
2 Cover plate
3 Substrate
4 Sample injection port
5 Heater unit
5a Heater
5b Heater
6 Detection electrode
7 Sample discharge port
8 Reaction channels
8a Reaction channel
8b Reaction channel
8c Reaction channel
8d Reaction channel
8e Reaction channel
9 Reaction reagent reservoir
10 Detecting section
11 Silicon oxide film
12 Silicon substrate
13 Oxide film
14 Channel
15 Glass plate
16 Metal
17 Metal thin film
18 Silicon oxide film
19 Detection electrode base

## Claims

1. A microfluidic device comprising a reaction channel;
wherein a plurality of thermal cycle regions which respectively comprise at least two thermal regions with different temperatures are repeatedly provided, and the reaction channel passes through the plurality of thermal cycle regions.

2. The microfluidic device according to claim 1, further comprising a heater unit,
wherein the heater unit comprises a plurality of heaters which are set at different temperatures, and
the thermal regions are formed around the heaters.

3. The microfluidic device according to claim 2, further comprising a substrate and a cover plate which are bonded to each other,
wherein the heater unit is placed on at least either one of the substrate and the cover plate, and
the reaction channel is provided between the substrate and the cover plate.

4. The microfluidic device according to any one of claims 1 to 3, wherein at least two of the reaction channels are arranged in parallel.

5. The microfluidic device according to claim 4, wherein the at least two of the reaction channels arranged in parallel share the same heater unit.

6. The microfluidic device according to claim 4 or 5, further comprising a sample injection port and a sample discharge port,
wherein the at least two of the reaction channels arranged in parallel are connected to the same sample injection port.

7. The microfluidic device according to any one of claims 2 to 6, wherein the heaters are metal thin film heaters.

8. The microfluidic device according to any one of claims 1 to 7, further comprising a detection electrode to measure a concentration of a reaction fluid flowing in the reaction channel.

9. The microfluidic device according to any one of claims 6 to 8, further comprising a reaction reagent which is held at a position between the sample injection port and the reaction channel which passes through the plurality of thermal cycle regions.

10. Microfluidic devices, wherein a plurality of the microfluidic devices according to any one of claims 1 to 9 are arranged in series or in parallel or in a combination thereof.
